# FASCICULE DE BREVET EUROPEEN

(11) **EP 0 600 999 B1**
(45) Date de publication et mention de la délivrance du brevet: **13.11.1996**
(21) Numéro de dépôt: 92918127.9
(22) Date de dépôt: 10.08.1992
(51) Int. Cl.: A61L 2/26, A23L 3/10, A23L 3/14

(54) **DISPOSITIF DE MAINTIEN D'OBJETS A L'INTERIEUR D'UN TAMBOUR TOURNANT**
HALTEVORRICHTUNG FÜR GEGENSTÄNDE IN EINER DREHTROMMEL
DEVICE FOR MAINTAINING OBJECTS INSIDE A ROTATING DRUM

(30) Priorité: 30.08.1991 FR 9110802
(43) Date de publication de la demande: 15.06.1994
(73) Titulaire: BARRIQUAND STERIFLOW, F-42300 Roanne (FR)
(72) Inventeur: ROUMAGNAC, Jean-Patrick, F-42120 Le Coteau (FR); NAVEROS, Francisco, F-42300 Roanne (FR); TIMMERMANS, Jacques, F-42120 Le Coteau (FR)
(74) Mandataire: Dronne, Guy
(86) Numéro de dépôt international: FR9200784
(87) Numéro de publication internationale: WO9304707

(56) Documents cités:
- DE-U- 7 905 136
- FR-A- 2 481 888

## Description

La présente invention a pour objet un dispositif de maintien d'objets à l'intérieur d'un tambour tournant.

De façon plus précise, l'invention concerne un dispositif qui permet de maintenir des objets disposés en pile à l'intérieur du tambour tournant d'appareils tels que des stérilisateurs, des autoclaves ou analogues.

La stérilisation d'objets ou de produits de l'industrie alimentaire ou pharmaceutique, tels que des boîtes, des bocaux, des barquettes, des flacons, etc..., est souvent réalisée dans des autoclaves dits discontinus à l'intérieur desquels on introduit les produits à traiter puis on les en extrait après l'achèvement du cycle de traitement prévu. Ces produits à traiter sont disposés dans des paniers, des plateaux empilables ou autres éléments de stockage similaires. Pour certains de ces produits, le traitement thermique de stérilisation doit être effectué avec une agitation du produit à l'intérieur de son emballage. Cette agitatation est produite par la rotation, à l'intérieur de l'autoclave, d'un tambour enfermant des paniers remplis des emballages avec les produits à stériliser qu'ils contiennent. Le même type de problème se pose lorsque l'on veut égoutter ou basculer certains produits dans un appareil autre qu'un autoclave qui est alors appelé égoutteur ou basculeur. Cette dernière opération permet l'élimination de l'eau déposée sur des produits par gravité lors de la stérilisation.

Le brevet français No. 86 02046 décrit en détails un exemple d'un tel autoclave de stérilisation à tambour tournant.

Lors de la rotation du tambour, il est bien sûr nécessaire d'éviter tout déplacement des emballages, ce qui pourraît entraîner leur endommagement ou leur destruction en les immobilisant à l'intérieur du tambour à l'aide d'un dispositif spécifique de maintien. En général, les emballages individuels des produits sont empilés dans des bacs ou placés dans des plateaux ou casiers et c'est l'ensemble de ces bacs ou analogues qu'il y a lieu de maintenir à l'intérieur du tambour.

Par simplification dans la suite de la présente description et dans les revendications, on désignera d'une manière générale par objet tout emballage de produit à stériliser.

Pour maintenir en place les objets empilés à l'intérieur du tambour tournant, on a déjà proposé, comme dans la demande de brevet français FR-A-2 605 226, de commander des plateaux de maintien des objets à l'aide de vérins à simple effet disposés à la partie supérieure du tambour et qui agissent perpendiculairement aux plateeux de maintien. Une telle solution permet certes d'obtenir le maintien des objets à l'intérieur du tambour sans intervention manuelle mais elle n'est pas satisfaisante dans la mesure où les vérins à simple effet doivent être associés à des ressorts de rappel pour permettre l'évacuation des plateaux de maintien. En outre, les tiges des vérins sont directement reliées aux plateaux presseurs, ce qui limite la course et impose des vérins de type particulier en raison du manque de place.

On a également proposé dans le brevet américain US-A-2 629 312 d'assurer le maintien d'objets à l'intérieur d'un tambour tournant à l'aide de mécanismes de déplacement d'un plateau de pression, ce mécanisme étant actionné manuellement à l'aide d'une manivelle et disposé à la partie supérieure du tambour. Cette solution n'est guère satisfaisante dans la mesure où le milieu dans lequel il faut intervenir est hostile et où ces interventions manuelles ne permettent pas la mise en place d'une chaîne de traitement automatique des objets.

En outre et surtout, dans toutes les solutions proposées, les mécanismes de pressage des plateaux sont disposés dans la partie supérieure du tambour. Cette solution ne permet qu'une course limitée du plateau et de plus, compte tenu de la place réduite disponible, elle ne permet pas d'assurer un serrage uniforme des plateaux.

Pour remédier aux inconvénients mentionnés ci-dessus, un objet de l'invention est de fournir un dispositif de maintien d'objets en piles dans un tambour tournant, notamment de stérilisateur ou d'appareil analogue, qui permet d'une part d'assurer un pressage efficace et également réparti sur l'ensemble des piles d'objets de façon automatique et qui, de plus, s'adapte mieux aux espaces disponibles entre la paroi du tambour et les piles d'objets à stériliser.

Pour atteindre ce but, le dispositif de maintien d'au moins une pile d'objets à l'intérieur d'un tambour tournant, ledit tambour comprenant un plancher pour recevoir ladite pile comprend au moins un plateau presseur pour être appliqué à la partie supérieure de ladite pile et des moyens de déplacement dudit plateau pour appliquer effectivement celui-ci sur ladite pile, et il se caractérise en ce que les moyens de déplacement comprennent au moins un organe formant levier disposé sur au moins un des côtés de ladite pile, monté pivotant autour d'un axe solidaire dudit tambour, parallèle au plancher, et perpendiculaire à l'axe de rotation dudit tambour, et des moyens de commande du pivotement dudit levier autour de son axe disposé également sur un des côtés de ladite pile, une première extrémité du levier étant entraînée par les moyens de commande et une deuxième extrémité étant solidaire dudit plateau, le déplacement de la première extrémité du levier dans un premier sens provoquant le rapprochement de la deuxième extrémité vers ledit plancher, par quoi le plateau est appliqué sur l'extrémité supérieure de ladite pile.

Selon un mode préféré de mise en oeuvre de l'invention, le tambour renferme une pluralité de piles d'objets et il se caractérise en ce qu'il comprend, pour chaque pile d'objets, deux paires de leviers identiques, chaque paire étant disposée d'un côté de ladite pile, lesdits moyens de commande de déplacement appliquant aux premières extrémités des leviers de chaque paire, un déplacement indépendant de celui qui est appliqué à une autre paire, par quoi le serrage de chaque plateau associé à chaque pile d'objets est réalisé indépendamment des autres plateaux.

Selon un premier mode de réalisation de l'invention, le pivotement des leviers est commandé à l'aide de vérins pneumatiques ou hydrauliques, chaque vérin commandant une paire de leviers.

Selon un deuxième mode de réalisation de l'invention, les déplacements des premières extrémités des leviers sont réalisés par des systèmes mécaniques, par exemple du type vis-écrou, l'indépendance de la commande de chaque paire de leviers étant de préférence assurée par l'interposition de systèmes élastiques.

D'autres caractéristiques de la présente invention apparaîtront plus clairement à la lecture de la description qui suit de plusieurs modes de réalisation de l'invention donnés à titre d'exemples non limitatifs. La description se réfère aux figures annexées sur lesquelles :
- la figure 1 est une vue en élévation en coupe verticale d'un premier mode de réalisation du système de maintien à commande par vérin ;
- la figure 2 est une vue de côté selon la flèche II de la figure 1 :
- la figure 3 est une vue en élévation et en coupe verticale d'un deuxième mode de réalisation du dispositif de maintien du type à commande mécanique :
- la figure 4 est une vue de côté du dispositif de la figure 3 selon la flèche IV de cette figure ;
- les figures 5 et 6 sont des vues partielles respectivement en élévation et de face d'une partie du mécanisme des figures 3 et 4 permettant le déplacement des moyens de maintien des piles d'objets.

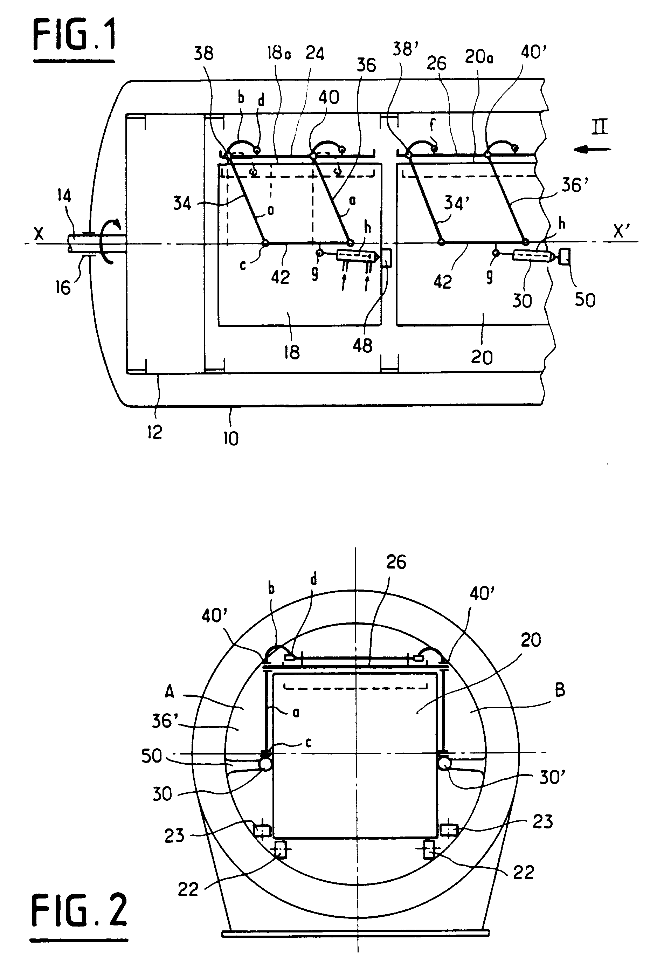

En se référant tout d'abord aux figures 1 et 2, on va décrire un premier mode de réalisation du système de maintien comportant une commande par vérin pneumatique ou hydraulique.

Sur ces figures, on a représenté schématiquement l'enceinte externe 10 de l'autoclave destinée à résister à la pression et qui est fermée par un couvercle non représenté et, à l'intérieur de l'enceinte 10, le tambour tournant 12 qui est monté rotatif autour de l'axe XX'. On a représenté de façon simplifiée une partie de l'arbre 14 traversant le palier 16 de l'enceinte externe 10 qui permet la mise en rotation du tambour 12 autour de son axe XX'. A l'intérieur du tambour 12, on a fait figurer deux empilements d'objets 18 et 20 représentés symboliquement par des rectangles. Comme on le voit mieux sur la figure 2, l'empilement 20 repose à sa partie inférieure sur une série de rouleaux 22 constituant un plancher du tambour et facilitant l'introduction des empilements d'objets à l'intérieur de celui-ci. En outre, des galets, tels que 23, assurent le centrage et le maintien latéral des empilements 18 à l'intérieur du tambour. Comme cela est bien connu, les empilements d'objets 18 et 20 sont maintenus contre le plancher par des plateaux presseurs 24 et 26. Dans la position de repos, les plateaux 24 et 26 sont relevés pour permettre le libre engagement des empilements d'objets alors qu'en position de travail les plateaux sont appliqués avec pression sur la partie supérieure 18a, 20a des empilements d'objets.

Comme on l'a déjà indiqué, selon une caractéristique essentielle de l'invention, les dispositifs de maintien des objets qui permettent de commander les déplacements des plateaux presseurs 24 et 26 entre leur position de repos et leur position active sont disposées de chaque côté des empilements d'objets, c'est-à-dire dans les zones latérales A et B du tambour 12. Chaque plateau presseur est commandé par un vérin pneumatique 30, 30' pour le plateau 26 et 32 (et 32') pour le plateau 24, chaque vérin étant disposé dans une des zones latérales A et B du tambour. Le vérin 32 est relié au plateau correspondant 24 par une paire de leviers référencés 34 et 36. On a bien sûr les mêmes paires de leviers de l'autre côté des piles d'objets 18 et 20. Chaque levier est monté pivotant autour d'un axe respectivement 38, 40 pour les leviers 34 et 36 et 38', 40' pour les leviers 34' et 36' associés à la pile d'objets 20. Chaque levier comporte un premier bras a qui s'étend depuis l'axe d'articulation associé jusqu'une deuxième extrémité c. Chaque levier comporte également un deuxième bras légèrement coudé b qui s'étend depuis l'articulation 38 jusqu'à une deuxième extrémité d. La deuxième extrémité d de chaque levier est articulée sur la face supérieure du plateau correspondant 24 ou 26. Les axes de pivotement 38, 40, 38' et 40' sont disposés parallèlement au plancher constitué par les rouleaux 22 et perpendiculairement à l'axe XX' de rotation du tambour et sont solidaires du tambour 12. En d'autres termes, les leviers se déplacent dans des plans perpendiculaires au plan vertical de la figure 1 ou de la figure 2. En outre, comme on le voit, les bras inférieurs a des leviers sont disposés dans les zones latérales A et B du tambour.

Comme le montre mieux la figure 1, le bras b de chaque levier fait, dans sa direction générale, avec le bras a correspondant un angle f qui est inférieur à 90°. Les extrémités c des bras a de chaque paire de leviers sont reliées entre elles par une tringle rigide 42 pour les leviers 34, 36 et 42' pour les leviers 34', 36'. L'extrémité g de la tige de chacun des vérins est articulée en un point de la tringle 42 associée à la paire de leviers correspondants. Le corps h de chaque vérin est monté pivotant sur un support référencé 48 pour le vérin 32 et 50 pour le vérin 30.

On comprend qu'en commandant les vérins 30, 32 et les vérins correspondants disposés dans l'autre partie latérale du tambour, on provoque le déplacement de la des tringles 42, 42' et donc le pivotement des bras a des paires de leviers autour des axes associés 38, 40, 38', 40', ce qui provoque simultanément l'abaissement des plateaux 24 et 26 contre les extrémités supérieures des piles d'objets 18 et 20. De préférence, les vérins 30, 32 sont du type à double effet permettant ainsi de commander de façon bidirectionnelle les déplacements de l'extrémité de la tige g dans un sens et dans l'autre pour permettre le soulèvement et l'abaissement des plateaux 24 et 26.

On voit que, grâce eu système qui vient d'être décrit, on utilise la place disponible dans les zones latérales A et D du tambour, ce qui permet d'utiliser des vérins de type classique présentant une course suffisante. En outre, on comprend que, par le jeu des longueurs des bras a et b des leviers, on obtient une démultiplication de l' effort, ce qui permet de réduire la puissance des vérins utilisés. En outre, on comprend que le débattement dans le sens vertical des extrémités d des bras b des leviers sont d'une faible amplitude, ce qui est intéressant puisque la place disponible dans la zone supérieure c du tambour est réduite comme on l'a déjà expliqué.

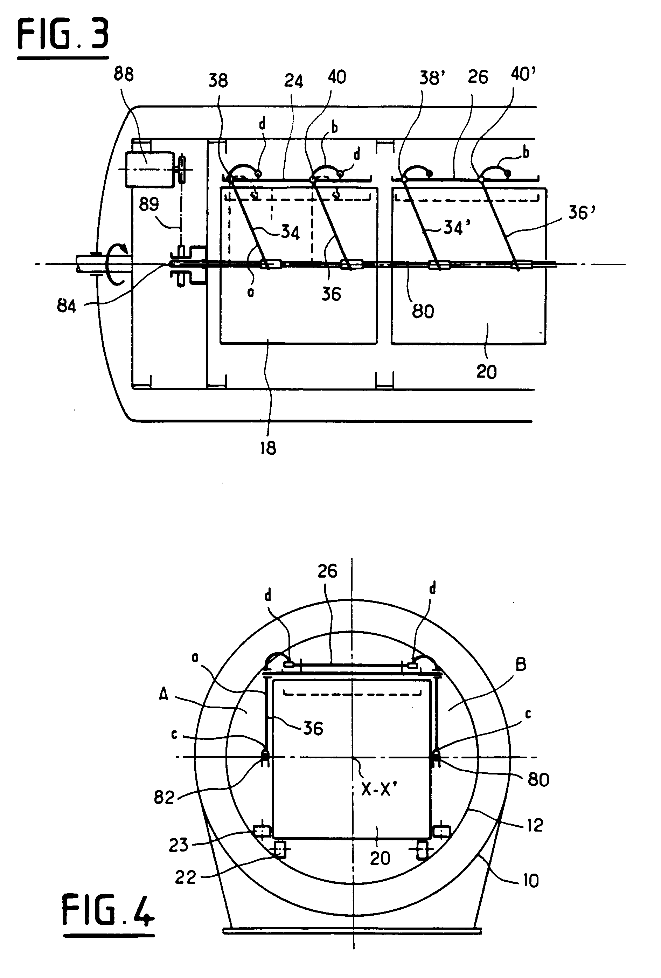

En se référant maintenant aux figures 3 à 6, on va décrire un deuxième mode de réalisation du dispositif de maintien des objets dans lequel la commande du déplacement des plateaux presseurs est réalisée de façon mécanique.

Sur ces figures, on a repris les références déjà utilisées en liaison avec les figuresd 1 et 2 pour les parties communes aux deux modes de réalisation. Comme on le voit sur le figure 3, dans ce mode de réalisation, les plateaux 24 et 26 sont déplacés par des paires de leviers 34, 36, 34', 36' identiques à ceux de la figure 1. Ces leviers comportent chacun deux bras a et b qui sont montés pivotant autour des axes 38, 40, 38', 40', ces leviers étant disposés dans les zones latérales A et B du tambour, c'est-à-dire de part et d'autre des empilements d'objets 18 et 20. Les extrémités d des bras b des leviers sont rendues solidaires des plateaux 24 et 26.

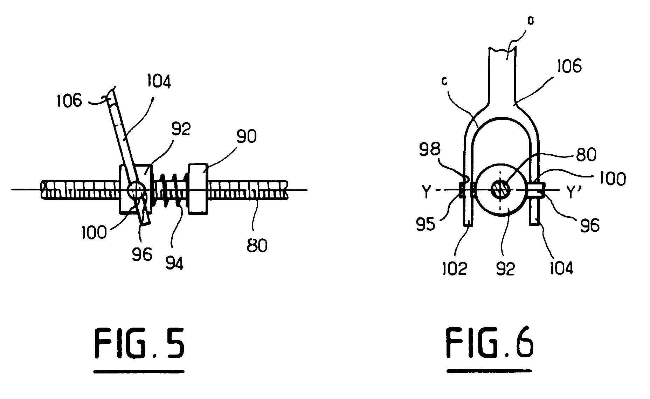

Ce mode de réalisation diffère de celui des figures 1 et 2 par la façon dont on commande les déplacements des extrémités des bras a des leviers afin de provoquer le soulèvement et l'abaissement des plateaux presseurs 24 et 26. Ce déplacement est commandé par deux tiges filetées 80 et 82 qui sont parallèles à l'axe de pivotement XX' du tambour et disposées respectivement dans les régions latérales A et B de celui-ci. Chaque tige filetée comporte une extrémité 84, respectivement 86, qui est reliée à un moteur d'entraînement 88 par l'intermédiaire d'un ensemble réducteur 90. Les tiges filetées 80 et 82 sont supportées par des paliers non représentés sur les figures. Pour chaque levier, la tige filetée 80, respectivement 82, comporte un écrou 90 comme le montrent les figures 5 et 6. Cet écrou 90 est associé à une bague 92 engagée librement sur la tige filetée 80, l'écrou 90 étant relié à la bague 92 par un ressort précontraint 94. Chaque bague 92 est munie de deux oreilles 95 et 96 montées libres en rotation autour d'un axe YY' perpendiculaire à la tige filetée 80. Les oreilles 95 et 96 sont munies de perçages 98 et 100 dans lesquels peuvent s'engager librement les branches 102 et 104 d'une fourche 106 qui constitue l'extrémité c du bras a de chaque levier 34, 36, 34', 36'. La mise en rotation de la tige 80 provoque le déplacement correspondant de l'écrou 90. Celui-ci à son tour transmet ce déplacement à la bague 92 par l'intermédiaire du ressort 94. Ce déplacement de la bague 92 entraîne lui-même le pivotement dans un sens convenable du bras a du levier associé et, en conséquence, le déplacement du plateau presseur 24 ou 26. On comprend que le système constitué par les oreilles 98, 94 et 96 dans lesquelles sont librement engagées les extrémités 102 et 104 de la fourche 106 permettent effectivement le pivotement des leviers sans introduire de contraintes. Il en ba bien sûr de même pour la tige filetée 82 et les paires de leviers disposées de l'autre côté des piles d'objets.

On comprend également que la liaison par ressort 94 entre l'écrou moteur 90 et la bague 92 entraînée permet un certain désaccouplement des pivotements respectifs des leviers 34, 36, 34', 36', ce qui permet ainsi d'adapter la position de maintien des plateaux 24, 26 à la hauteur effective des empilements d'objets 18, 20. On comprend également que chaque levier d'une même paire, par exemple les leviers 34 et 36, étant également entraîné par l'intermédiaire des systèmes élastiques 94, cela permet d'assurer un pressage correct du plateau même si la pile d'objets n'a pas en tous points exactement la même hauteur.

Il découle de la description précédente que la commande des plateaux presseurs qui est reportée dans les parties latérales du tambour, de part et d'autre des piles d'objets, présente de nombreux avantages par rapport aux solutions connues dans lesquelles les moyens de pressage sont disposés à la partie supérieure du tambour, dans le plan médian de celui-ci. Les contraintes dues au pressage des plateaux sont reportées directement à le partie inférieure du tambour au lieu d'être reportées au "sommet" de celui-ci. Le dégagement de la partie supérieure du tambour au-dessus des piles d'objets facilite l'aspersion des objets lorsque l'autoclave fonctionne en "statique". Les efforts de pressage sur les plateaux sont appliqués aux quatre "sommets" de ceux-ci, ce qui tend à uniformiser les contraintes. Enfin dans le cas de la commande par vérins, on peut utiliser des vérins classiques avec diamètre et course standard.

## Revendications

1. Dispositif de maintien d'au moins une pile d'objets (18, 20) à l'intérieur d'un tambour tournant (12), ledit tambour comprenant un plancher (22) pour recevoir ladite pile, ledit dispositif de maintien comprenant au moins un plateau presseur (24, 26) pour être appliqué à la partie supérieure de ladite pile et des moyens de déplacement dudit plateau pour appliquer effectivement celui-ci sur ladite pile, se caractérise en ce que les moyens de déplacement comprennent au moins un organe formant levier (34, 36, 34', 36') disposé sur au moins un des côtés (A, B) de ladite pile, monté pivotant autour d'un axe (38, 40, 38', 40') solidaire dudit tambour parallèle au plancher et perpendiculaire à l'axe de rotation (XX') dudit tambour (12) et des moyens de commande (30, 32, 80, 82) du pivotement dudit levier autour de son axe, disposés également sur un des côtés de ladite pile, une première extrémité (C) du levier étant entraînée par les moyens de commande et une deuxième extrémité (d) étant solidaire dudit plateau (24, 26), le déplacement de la première extrémité du levier dans un premier sens provoquant l'abaissement de la deuxième extrémité vers ledit plancher, par quoi le plateau est appliqué sur l'extrémité supérieure de ladite pile.

2. Dispositif de maintien selon la revendication 1, pour maintenir une pluralité de piles d'objets (18, 20), caractérisé en ce qu'il comprend pour chaque pile d'objets deux paires de leviers (34, 36, 34', 36') identiques, chaque paire de leviers étant disposée d'un côté (A, B) de ladite pile, lesdits moyens de commande appliquant aux premières extrémités (c) des leviers de chaque paire un déplacement indépendant de celui qui est appliqué à une autre paire par quoi le serage de chaque plateau associé à chaque pile d'objets est réalisé indépendamment des autres plateaux.

3. Dispositif selon la revendication 2, caractérisé en ce que chaque levier (34, 36, 34', 36') comprend un premier bras (a) et un deuxième bras coudé (b) disposés de part et d'autre de son axe de pivotement (38, 40, 38', 40'), l'angle a entre le segment joignant l'axe de pivotement à ladite première extrémité (c) du levier et le segment joignant l'axe de pivotement à la deuxième extrémité (d) du levier est inférieur à 90°.

4. Dispositif selon l'une quelconque des revendications 2 et 3, caractérisé en ce que les moyens de commande du pivotement de chaque paire de leviers (34, 36, 34', 36') comprennent un vérin (30, 32, 30', 32') dont l'extrémité libre (g) de la tige est reliée à la première extrémité (c) desdits leviers.

5. Dispositif selon la revendication 4, caractérisé en ce que les moyens de commande du pivotement comprennent en outre une barre (42) relient entre elles les premières extrémités (c) des leviers d'une même paire, ladite barre étant reliée à ladite extrémité libre (g) de la tige du vérin (30, 32, 30', 32').

6. Dispositif selon l'une quelconque des revendications 2 et 3, caractérisé en ce que la première extrémité (c) de chacun des leviers (34, 36, 34', 36') disposés d'un même côté des piles d'objets est reliée mécaniquement à une pièce (90) entraînée en translation parallèlement audit axe de rotation du tambour par lesdits moyens de commande (80, 82).

7. Dispositif selon la revendication 6, caractérisé en ce que la première extrémité (c) de chaque levier est reliée à ladite pièce (90) par un système élastique (92, 94) déformable selon la direction de déplacement de ladite pièce.

8. Dispositif selon l'une quelconque des revendications 6 et 7, caractérisé en ce que chaque pièce (90) est un écrou coopérant avec une tige filetée (80, 82) disposée parallèlement audit axe de rotation (XX') du tambour, tous les écrous disposés d'un même côté des piles d'objets coopérant avec une même tige filetée.

## Claims

1. System for supporting at least one stack of objects (18, 20) inside a rotating drum (12), said drum comprising a floor (22) to receive said stack, said support system comprising at least one presser plate (24, 26) for application against the top part of said stack and means for displacing said plate so as to effectively apply it against said stack, the system being characterized in that the displacement means comprise at least one member forming a lever (34, 36, 34', 36') disposed on at least one side (A, B) of said stack, and pivotally mounted about a pin (38, 40, 38', 40') secured to said drum, parallel to the floor and perpendicular to the axis of rotation (XX') of said drum (12), and means (30, 32, 80, 82) for controlling the pivoting of said lever about its pin, said control means also being disposed on one side of said stack, a first end (c) of the lever being driven by the control means and a second end (d) being secured to said plate (24, 26), displacement of the first end of the lever in a first direction causing the second end to be lowered down towards said floor, whereby the plate is applied against the top end of said stack.

2. Support device according to claim 1, for supporting a plurality of stacks of objects (18, 20), characterized in that it comprises, for each stack of objects, two pairs of identical levers (34, 36, 34', 36'), each pair being disposed on one side (A, B) of said stack, said control means applying to the first ends (c) of the levers of each pair, a displacement which is independent of that which is applied to another pair, whereby the clamping of each plate associated with each stack of objects is achieved independently of the other plates.

3. Device according to claim 2, characterized in that each lever (34, 36, 34', 36') comprises a first arm (a) and a second arm (b) disposed on either side of its pivot pin (38, 40, 38', 40'), the angle (a) between the segment joining the pivot pin to said first end (c) of the lever and the segment joining the pivot pin to the second end (d) of the lever is less than 90°.

4. Device according to any one of claims 2 and 3, characterized in that the means for controlling the pivoting of each pair of levers (34, 36, 34', 36') comprises an actuator (30, 32, 30', 32'), the free end (g)of whose rod is connected to the first end (c) of a said levers.

5. Device according to claim 4, characterized in that the means for controlling the pivoting further comprises a bar (42) connecting together the first ends (c) of the levers of a single pair, said bar being connected to said free end (g) of the rod of the actuator (30, 32, 30', 32').

6. Device according to any one of claims 2 and 3, characterized in that the first end (c) of each of the levers (34, 36, 34', 36') arranged on the same side of the stacks of objects is mechanically connected to a part (90) which is moved in translation parallel to said axis of rotation of the drum, via said control means (80, 82).

7. Device according to claim 6, characterized in that the first end (c) of each lever is connected to said part (90) by a resilient system (92, 94) which is deformable along the direction of displacement of said part.

8. Device according to any one of claims 6 and 7, characterized in that each part (90) is a nut co-operating with a threaded rod (80, 82) disposed parallel to said his of rotation (XX') of the drum, all the nuts disposed on the same side of the stacks of objects co-operating with the same threaded rod.

## Patentansprüche

1. Vorrichtung zur Halterung von mindestens einem Stapel (18, 20) von Gegenständen im Inneren einer Drehtrommel (12), wobei die Trommel einen Boden (22) zur Aufnahme des Stapels aufweist und die Halterungsvorrichtung mindestens eine auf den oberen Teil des Stapels zu pressende Druckplatte (24, 26) und Einrichtungen zum Verschieben der Platte, um diese wirkungsvoll auf den Stapel zu drücken, aufweist, dadurch gekennzeichnet, daß die Verschiebeeinrichtungen mindestens ein einen Hebel (34, 36, 34', 36') bildendes Organ, welches auf mindestens einer Seite (A, B) des Stapels schwenkbar um eine mit der Trommel fest verbundene Achse (38, 40, 38', 40') parallel zum Boden und senkrecht zur Rotationsachse (XX') der Trommel (12) angeordnet ist, und Einrichtungen (30, 32, 80, 82) zur Steuerung der Schwenkbewegung des Hebels um seine Achse, welche ebenfalls auf einer Seite des Stapels angeordnet sind, aufweisen, wobei ein erstes Ende (c) des Hebels von den Steuereinrichtungen angetrieben und ein zweites Ende (d) mit der Platte (24, 26) fest verbunden ist und die Verschiebung des ersten Endes des Hebels in einer ersten Richtung die Absenkung des zweiten Endes zum Boden hin bewirkt, wodurch die Platte auf das obere Ende des Stapels gedrückt wird.

2. Halterungsvorrichtung nach Anspruch 1 zur Halterung einer Mehrzahl von Stapeln (18, 20) von Gegenstanden, dadurch gekennzeichnet, daß sie für jeden Stapel von Gegenständen zwei Paare von identischen Hebeln (34, 36, 34', 36') aufweist, von denen jedes Paar Hebel auf einer Seite (A, B) des Stapels angeordnet ist, wobei die Steuereinrichtungen auf die ersten Enden (c) der Hebel jedes Paares eine Verschiehebewegung aufdrücken, die unabhängig von jener ist, die auf ein anderes Paar aufgedrückt wird, wodurch die Klemmung jeder jeweils einem Stapel von Gegenständen zugeordneten Platte unabhängig von den anderen Platten erfolgt.

3. Vorrichtung nach Anspruch 2, dadurch gekennzeichnet, daß jeder Hebel (34, 36, 34', 36') einen ersten Arm (a) und einen zweiten, gekrümmten Arm (b) aufweist, welche beiderseits der Schwenkachse (38, 40, 38', 40') desselben angeordnet sind, wobei der Winkel a zwischen dem die Schwenkachse mit dem ersten Ende (c) des Hebels verbindenden Abschnitt und dem die Schwenkachse mit dem zweiten Ende (d) des Hebels verbindenden Abschnitt kleiner als 90° ist.

4. Vorrichtung nach einem der Ansprüche 2 und 3, dadurch gekennzeichnet, daß die Einrichtungen zur Steuerung der Schwenkbewegung jedes Paares von Hebeln (34, 36, 34', 36') einen Stelltrieb (30, 32, 30', 32') aufweisen, dessen freies Stangenende (g) mit dem ersten Ende (c) der Hebel verbunden ist.

5. Vorrichtung nach Anspruch 4, dadurch gekennzeichnet, daß die Einrichtungen zur Steuerung der Schwenkbewegung weiters eine Stange (42) aufweisen, welche die ersten Enden (c) der Hebel ein- und desselben Paares miteinander verbindet, wobei die Stange mit dem freien Ende (g) der Stange des Stelltriebs (30, 32, 30', 32') verbunden ist.

6. Vorrichtung nach einem der Ansprüche 2 und 3, dadurch gekennzeichnet, daß das erste Ende (c) jedes der auf ein- und derselben Seite der Stapel von Gegenständen angeordneten Hebels (34, 36, 34', 36') mechanisch mit einem Stück (90) verbunden ist, welches von den Steuereinrichtungen (80, 82) in eine Translationsbewegung parallel zur Rotationsachse der Trommel versetzt wird.

7. Vorrichtung nach Anspruch 6, dadurch gekennzeichnet, daß das erste Ende (c) jedes Hebels durch ein je nach der Verschieberichtung des Stücks verformbares elastisches System (92, 94) mit dem Stück (90) verbunden ist.

8. Vorrichtung nach einem der Ansprüche 6 und 7, dadurch gekennzeichnet, daß jedes Stück (90) eine Mutter ist, die mit einer Gewindestange (80, 82) zusammenwirkt, welche parallel zur Rotationsachse (XX') der Trommel angeordnet ist, wobei sämtliche auf ein- und derselben Seite der Stapel von Gegenständen angeordnete Muttern mit ein- und derselben Gewindestange zusammenwirken.
